(19) **Europäisches Patentamt / European Patent Office / Office européen des brevets**

(11) **EP 3 360 577 A1**

(12) **EUROPEAN PATENT APPLICATION**
published in accordance with Art. 153(4) EPC

(43) Date of publication:
**15.08.2018 Bulletin 2018/33**

(21) Application number: **16853714.0**

(22) Date of filing: **06.10.2016**

(51) Int Cl.:
*A61K 47/24* (2006.01)　　*A61K 9/127* (2006.01)
*A61K 31/337* (2006.01)　　*A61K 31/357* (2006.01)
*A61K 31/4545* (2006.01)　*A61K 31/7048* (2006.01)
*A61K 39/395* (2006.01)　　*A61K 45/00* (2006.01)
*A61K 47/10* (2017.01)　　*A61K 47/28* (2006.01)
*A61K 47/34* (2017.01)　　*A61P 35/00* (2006.01)

(86) International application number:
**PCT/JP2016/079837**

(87) International publication number:
**WO 2017/061562 (13.04.2017 Gazette 2017/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.10.2015　JP 2015199292**

(71) Applicants:
• **Ensuiko Sugar Refining Co., Ltd.**
**Tokyo 103-0012 (JP)**
• **Hamada, Hiroki**
**Okayama 709-0827 (JP)**
• **Shimizu, Yoshio**
**Higashi-ku**
**Okayama-shi**
**Okayama 709-0841 (JP)**
• **National University Corporation Okayama University**
**Okayama-shi, Okayama 700-8530 (JP)**

(72) Inventors:
• **HAMADA, Hiroki**
**Akaiwa-shi**
**Okayama 709-0827 (JP)**
• **SENO, Masaharu**
**Okayama-shi**
**Okayama 700-8530 (JP)**
• **KASAI, Tomonari**
**Okayama-shi**
**Okayama 700-8530 (JP)**
• **SHIGEHIRO, Tsukasa**
**Okayama-shi**
**Okayama 700-8530 (JP)**
• **HARA, Koji**
**Tokyo 103-0012 (JP)**
• **ITO, Tetsuya**
**Tokyo 103-0012 (JP)**
• **FUJIWARA, Ichiro**
**Okayama-shi**
**Okayama 7038255 (JP)**

(74) Representative: **Vossius & Partner Patentanwälte Rechtsanwälte mbB**
**Siebertstrasse 3**
**81675 München (DE)**

(54) **LIPOSOME INCLUDING TAXANE COMPOUND**

(57)　An object of the present invention is to provide a method for encapsulating a poorly water-soluble pharmacologically active substance in a liposome with high efficiency.

The present invention provides a composition comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

EP 3 360 577 A1

[Fig 1]

[Fig 1]

**Description**

Technical Field

The present invention relates to a liposome encapsulating a taxane compound.

Background Art

Taxane compounds such as paclitaxel or docetaxel have superior anti-cancer activity; on the other hand, taxane compounds have disadvantages, including poor water solubility. Therefore, taxane compounds are administered to cancer patients by being dissolved in ethanol containing a surfactant such as Cremophor.

However, a new problem of such surfactants, significant side effects on humans, has recently been attracting attention. In light of this problem, development of a liposome encapsulating a taxane compound, and use of the liposome as a DDS formulation, have been attempted.

Specifically, a method of encapsulating a taxane compound in a liposome by a remote loading method, which utilizes the principle of solubility gradient, has been known, as disclosed in PTL 1.

Another known method is the method disclosed in NPL 1 using a passive loading method, comprising containing in advance a taxane compound in a lipid bilayer membrane for use in liposome production, and forming a liposome using the surfactant as mentioned above.

Citation List

Patent Literature

PTL 1: WO2013/141346

Non-patent Literature

NPL 1: Tao et al., Int. J. Pharm; 338(2007)317-326

Summary of Invention

Technical Problem

In the method of PTL 1, the efficiency in encapsulating a poorly water-soluble pharmacologically active substance in a liposome is very low. Further, with regard to the lipid bilayer membrane containing a poorly water-soluble pharmacologically active substance disclosed in NPL 1, the present inventors confirmed that contact of the lipid bilayer membrane with a surfactant, such as Cremophor, in an aqueous solvent did not even form a liposome. An object of the present invention is to provide a method for encapsulating a poorly water-soluble pharmacologically active substance in a liposome with high efficiency.

Solution to Problem

As a result of extensive research to solve the above problems, the present inventors discovered that a poorly water-soluble pharmacologically active substance may be efficiently encapsulated in a liposome by using a composition containing specific components, as detailed below.

The present invention was accomplished based on the above findings, and broadly includes the following embodiments.

Item 1 A composition comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

Item 2 The composition according to Item 1, wherein the lipid having a phosphatidylcholine group is at least one member selected from the group consisting of hydrogenated soy lecithin (HSPC), egg yolk phospholipid (EPC), distearoyl phosphatidylcholine (DSPC), dimyristoyl phosphatidylcholine (DMPC), dipalmitoyl phosphatidylcholine (DPPC), and dioleoyl phosphatidylcholine (DOPC).

Item 3 The composition according to Item 1 or 2, wherein the cholesterol compound is at least one member selected from the group consisting of cholesterol, cholestanol, 7-dehydrocholesterol, and phytosterol.

Item 4 The composition according to any one of Items 1 to 3, wherein the lipid having a phosphatidylethanolamine group is at least one member selected from the group consisting of distearoyl phosphatidylethanolamine (DSPE), dipalmitoyl phosphatidylethanolamine (DPPE), dimyristoyl phosphatidylethanolamine (DMPE), and dioleoyl phosphatidylethanolamine (DOPE).

Item 5 The composition according to any one of Items 1 to 4, wherein any one of the lipid having a phosphatidylethanolamine group, the cholesterol compound, and the lipid having a phosphatidylcholine group is a lipid modified by polyalkylene glycol.

Item 6 The composition according to any one of Items 1 to 5, wherein the poorly water-soluble pharmacologically active substance is at least one member selected from the group consisting of taxane compound, macrolide compound, vinca alkaloid compound, quinoline alkaloid compound, and etoposide compound.

Item 7 The composition according to Item 6, wherein the taxane compound is at least one member selected from the group consisting of paclitaxel, docetaxel, cabazitaxel, and glycosides thereof.

Item 8 The composition according to Item 6, wherein the macrolide compound is at least one member selected from the group consisting of bafilomycin, concanamycin, azithromycin, and clarithromycin.

Item 9 The composition according to any one of Items 1 to 8, wherein the composition is used to form a lipid film.

Item 10 A lipid film comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

Item 11 A method for producing a liposome encapsulating a poorly water-soluble pharmacologically active substance, comprising the step of bringing the lipid film of Item 10 into contact with a polyoxyethylene ester compound in an aqueous solvent.

Item 12 The method according to Item 11, wherein lower alcohol is further contained as an aqueous solvent.

Item 13 The method according to Item 11 or 12, wherein a buffer is further contained as an aqueous solvent.

Item 14 The method according to any one of Items 11 to 13, further comprising the step of loading an antibody recognizing a cancer cell.

Item 15 A liposome formulation comprising a liposome encapsulating a lipid having a phosphatidylcholine group, a cholesterol compound, a lipid having a phosphatidylethanolamine group, a poorly water-soluble pharmacologically active substance, and a polyoxyethylene ester compound, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

I. Composition

Composition (I) encompasses the inventions according to the embodiments described in the following Items (I-1) to (I-9).

Item (I-1)

A composition comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

Item (I-2)

The composition according to Item (I-1), wherein the lipid having a phosphatidylcholine group is at least one member selected from the group consisting of hydrogenated soy lecithin (HSPC), egg yolk phospholipid (EPC), distearoyl phosphatidylcholine (DSPC), dimyristoyl phosphatidylcholine (DMPC), dipalmitoyl phosphatidylcholine (DPPC), and dioleoyl phosphatidylcholine (DOPC).

Item (I-3)

The composition according to Item (I-1) or (I-2), wherein the cholesterol compound is at least one member selected from the group consisting of cholesterol, cholestanol, 7-dehydrocholesterol, and phytosterol.

Item (I-4)

The composition according to any one of Items (I-1) to (I-3), wherein the lipid having a phosphatidylethanolamine group is at least one member selected from the group consisting of distearoyl phosphatidylethanolamine (DSPE), dipalmitoyl phosphatidylethanolamine (DPPE), dimyristoyl phosphatidylethanolamine (DMPE), and dioleoyl phosphatidylethanolamine (DOPE).

Item (I-5)

The composition according to any one of Items (I-1) to (I-4), wherein any one of the lipid having a phosphatidylethanolamine group, the cholesterol compound, and the lipid having a phosphatidylcholine group is modified by polyalkylene glycol.

Item (I-6)

The composition according to any one of Items (I-1) to (I-4), wherein the poorly water-soluble pharmacologically active substance is at least one member selected from the group consisting of taxane compound, macrolide compound, vinca alkaloid compound, quinoline alkaloid compound, and etoposide compound.

Item (I-7)

The composition according to Item (I-6), wherein the taxane compound is at least one member selected from the group consisting of paclitaxel, docetaxel, cabazitaxel, and glycosides thereof.

Item (I-8)

The composition according to Item (I-6), wherein the macrolide compound is at least one member selected from the group consisting of bafilomycin, concanamycin, azithromycin, and clarithromycin.

Item (I-9)

The composition according to any one of Items (I-1) to (I-8), wherein the composition is used to form a lipid film.

II. Lipid Film

Lipid film (II) encompasses the invention according to the embodiment described in the following Item (II-1).

Item (II-1)

A lipid film comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

III. Method for Producing a Liposome Encapsulating a Poorly Water-soluble Pharmacologically Active Substance

The Method for producing a liposome encapsulating a poorly water-soluble pharmacologically active substance (III) encompasses the inventions according to the embodiments described in the following Items (III-1) to (III-4).

Item (III-1)

A method for producing a liposome encapsulating a poorly water-soluble pharmacologically active substance, comprising the step of bringing lipid film (II) into contact with a polyoxyethylene ester compound in an aqueous solvent.

EP 3 360 577 A1

Item (III-2)

The method according to Item (III-1), wherein lower alcohol is further contained as an aqueous solvent.

Item (III-3)

The method according to Item (III-1) or (III-2), wherein a buffer is further contained as an aqueous solvent.

Item (III-4)

The method according to any one of Items (III-1) to (III-3), wherein the method further comprising the step of loading an antibody recognizing a cancer cell.

IV. Liposome Formulation

Liposome formulation (IV) encompasses the inventions according to the embodiments described in the following Items (IV-1) to (IV-6).

Item (IV-1)

A liposome formulation comprising a liposome encapsulating a lipid having a phosphatidylcholine group, a cholesterol compound, a lipid having a phosphatidylethanolamine group, a poorly water-soluble pharmacologically active substance, and a polyoxyethylene ester compound, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

Item (IV-2)

The liposome formulation according to Item (IV-1), wherein lower alcohol is further contained in the liposome as an aqueous solvent.

Item (IV-3)

The liposome formulation according to Item (IV-1) and (IV-2), wherein a buffer is further contained in the liposome as an aqueous solvent.

Item (IV-4)

The liposome formulation according to any one of Items (VI-1) to (VI-3), wherein the liposome further carries an antibody recognizing a cancer cell.

Item (IV-5)

The liposome formulation according to any one of Items (VI-1) to (VI-4), wherein the liposome is a liposome produced by the method according to Method for producing a liposome encapsulating a poorly water-soluble pharmacologically active substance (III).

Item (IV-6)

The liposome formulation according to any one of Items (VI-1) to (VI-5), for use in the treatment or prevention of cancer.

Advantageous Effects of Invention

The composition of the present invention is preferably used when producing a liposome encapsulating a poorly water-soluble pharmacologically active substance.
The liposome formulation of the present invention exhibits an effect of alleviating side effects.

6

Brief Description of Drawings

Fig. 1 is a graph showing test results regarding encapsulation efficiency (EE:%) and loading efficiency (LE:%) of PTX-L(A) and gPTX-L(B) (N=4, * represents P<0.05). The horizontal axis indicates the molar ratio values (x) of PTX and gPTX added to the liposome relative to the total.

Fig. 2 is a graph showing test results regarding encapsulation efficiency (EE:%) and loading efficiency (LE:%) of DTX-L (N=4, * represents P<0.05). The horizontal axis indicates the molar ratio value (x) of DTX added to the liposome relative to the total.

Fig. 3 is a graph showing test results regarding physical property evaluation (particle size: nm, polydispersity index, and zeta potential: mV) of PTX-L(A) and gPTX-L(B) (N=4). The horizontal axis indicates the molar ratio values (x) of PTX and gPTX added to the liposome relative to the total.

Fig. 4 is a graph showing test results regarding physical property evaluation (particle size: nm, polydispersity index, and zeta potential: mV) of DTX-L (N=3). The horizontal axis indicates the molar ratio value (x) of DTX added to the liposome relative to the total.

Fig. 5 is a graph showing test results regarding retention rate (%) and particle size (nm) of PTX-L(A) and gPTX-L(B) (N=3). The horizontal axis indicates the molar ratio values (x) of PTX and gPTX added to the liposome relative to the total.

Fig. 6 is a graph showing test results regarding retention rate (%) and particle size (nm) of DTX-L (N=3). The horizontal axis indicates the molar ratio value (x) of DTX added to the liposome relative to the total.

Fig. 7 is a graph showing test results regarding anti-cancer activity evaluation ($IC_{50}$ value: nM) of PTX-L(A) and gPTX-L(B) (N=5, * represents P<0.05).

Fig. 8 is a graph showing test results regarding anti-cancer activity evaluation ($IC_{50}$ value: nM) of DTX-L.

Fig. 9 is a graph showing test results regarding encapsulation efficiency (EE:%), loading efficiency (LE:%), and physical property evaluation (particle size: nm, polydispersity index, and zeta potential: mV) of BafA1-L (N=3, * represents P<0.05). The horizontal axis indicates the molar ratio value (x) of bafilomycin added to the liposome relative to the total.

Fig. 10 is a graph showing test results regarding anti-cancer activity evaluation ($IC_{50}$ value: nM) of BafA1-L (N=5, * represents P<0.05).

Fig. 11 show photographic images of the results of a comparative test example. Image A shows a liposome produced based on the lipid film of the present invention, and image B shows a liposome produced based on the lipid film having the formulation disclosed in NPL 1.

Fig. 12 is a Kaplan-Meier diagram showing the results of a toxicity test using a docetaxel-encapsulated liposome described in Example 3 of the present invention. The vertical axis indicates the survival rate (%). The horizontal axis indicates the number of days after administration.

Description of Embodiments

In this specification, the term "lipid" means a simple lipid, complex lipid, derived lipid, or the like, but is not particularly limited to these lipids. Further, the "lipid" includes lipids modified by a polymer or the like, such as those modified by polyalkylene glycol.

(I) Composition

Composition (I) of the present invention is a composition comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

Composition (I) of the present invention is more preferably a composition in which the molar ratio of the lipid having a phosphatidylcholine group, the cholesterol compound, the lipid having a phosphatidylethanolamine group, and the poorly water-soluble pharmacologically active substance is 4 to 8:2 to 6:0.2 to 2:0.01 to 4.

Composition (I) of the present invention is further preferably a composition in which the molar ratio of the lipid having a phosphatidylcholine group, the cholesterol compound, the lipid having a phosphatidylethanolamine group, and the poorly water-soluble pharmacologically active substance is 5 to 7:3 to 5:0.3 to 1.5:0.05 to 3.

Composition (I) of the present invention is most preferably a composition in which the molar ratio of the lipid having a phosphatidylcholine group, the cholesterol compound, the lipid having a phosphatidylethanolamine group, and the poorly water-soluble pharmacologically active substance is 5 to 6:4 to 5:0.5 to 1:0.1 to 2.

The lipid having a phosphatidylcholine group is not particularly limited. Examples include phospholipids, more specifically, HSPC, ECP, DMPC, DPPC, DOPC, and the like. Among these, hydrogenated soy lecithin (HSPC) is preferable. These lipids having a phosphatidylcholine group may be used individually or in an appropriate combination of two or more kinds.

The cholesterol compound is not particularly limited. Examples include cholesterol, cholestanol, 7-dehydrocholesterol, phytosterol, and the like. Among these, cholesterol is preferable. These cholesterol compounds may be used individually or in an appropriate combination of two or more kinds.

The lipid having a phosphatidylethanolamine group is not particularly limited. Examples include phospholipids, more specifically, DSPE, DPPE, DMPE, DOPE, and the like. Among these, DSPE is preferable. These lipids, which have a phosphatidylethanolamine group, may be used individually or in an appropriate combination of two or more kinds.

Any of the lipids having a phosphatidylcholine group, the cholesterol compounds, and the lipids having a phosphatidylethanolamine group may be modified by polyalkylene glycol.

Polyalkylene glycol is not particularly limited. Examples of polyalkylene glycol include polyethylene glycol, polypropylene glycol, and the like. Among these, polyethylene glycol is preferable.

The molecular weight of the polyalkylene glycol is not particularly limited. The molecular weight is, for example, 500 to 3000 in number-average molecular weight. The molecular weight is, for example, 500 to 10000 in weight-average molecular weight.

The form of the modification by polyalkylene glycol is not particularly limited. Examples of the modification form include chemical bonds. In particular, the lipids having a phosphatidylcholine group and the lipids having a phosphatidylethanolamine group are preferably modified by a chemical bond of polyalkylene glycol, which is, however, not to a lipophilic group thereof but to a hydrophilic group (alcohol derivative group). Specific examples include phospholipids modified by polyethylene glycol, such as mPEG-DSPE.

The poorly water-soluble pharmacologically active substance is not particularly limited. For example, a pharmacologically active substance having significantly low water solubility may be used. The water solubility at 0°C is 10000 mg/L or less, preferably 1000 mg/L or less. Obviously, the lower limit of the solubility is 0 or more. Specific examples of such a poorly water-soluble pharmacologically active substance include taxane compounds, macrolide compounds, vinca alkaloid compounds, quinoline alkaloid compounds, etoposide compounds, and the like.

The taxane compounds are not particularly limited. Examples of taxane compounds include paclitaxel, docetaxel, cabazitaxel, and glycosides thereof.

The glycosides are not particularly limited. Known glycosides may be used. More specifically, glycosides modified by a monosaccharide, such as glucose or galactose, may be used. Among these, glucosides are preferable. Further, cyclic sugars such as pyranose or furanose are preferable in terms of the shapes of monosaccharides modifying the glycoside. Glucopyranoside is preferable as the glycoside of the present invention.

The glycoside may be modified by a sugar while having an appropriate group in its aglycone. For example, 7-glucosyloxyacetylpaclitaxel, in which an oxyacetyl group is present between paclitaxel and glucopyranoside, is most preferable.

The macrolide compounds are also not particularly limited. Examples of the macrolide compound include bafilomycin, bafilomycin, concanamycin, azithromycin, clarithromycin, and the like.

Further, the quinoline alkaloid compounds are not particularly limited. Examples of the quinoline alkaloid compound include camptothecin, irinotecan, and the like.

Further, the vinca alkaloid compounds are not particularly limited. Examples of the vinca alkaloid compound include vincristine, vinblastine, vindesine, vinorelbine, and the like.

Further, the etoposide compounds are not particularly limited. Examples of the etoposide compound include etoposide, teniposide, and the like.

Composition (I) of the present invention may be dissolved in a known solvent. The solvent is not particularly limited. For example, the solvent may be prepared from an organic solvent such as chloroform, and, if necessary, by mixing it with alcohol such as methanol or ethanol.

Composition (I) of the present invention may be preferably used to form lipid film (II) described below.

(II) Lipid Film

Lipid film (II) of the present invention may have the same constitution as that of composition (I) described above. More specifically, an embodiment having the same constitution as that of composition (I) described above may be encompassed as an embodiment of the lipid film of the present invention.

More specifically, lipid film (II) of the present invention is a lipid film comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

For example, as described above, when composition (I) is dissolved in a known solvent, lipid film (II) may be obtained by evaporating the solution to dryness.

The method of the evaporation to dryness is not particularly limited; for example, a method using an evaporator or the like may be used. The conditions of the evaporation to dryness are not particularly limited, and may be set within a range in which a lipid multilayer membrane, such as a lipid bilayer membrane, can be formed.

Lipid film (II) thus obtained may be preferably used as a raw material in the following method for producing a liposome encapsulating a poorly water-soluble pharmacologically active substance.

(III) Method for Producing a Liposome Encapsulating a Poorly Water-soluble Pharmacologically Active Substance

Method for producing a liposome encapsulating a poorly water-soluble pharmacologically active substance (III) of the present invention comprises the step of bringing lipid film (II) described above into contact with a polyoxyethylene ester compound in an aqueous solvent.

The term "encapsulating" in this specification is not particularly limited. Examples of the encapsulation include an embodiment in which a poorly water-soluble pharmacologically active substance is completely encapsulated in a liposome, and an embodiment in which a part of the molecules of the poorly water-soluble pharmacologically active substance penetrates the lipid multilayer membrane constituting the liposome.

The term "contact" in this specification is not particularly limited. Examples of the contact state include an embodiment of mixing lipid film (II) with an aqueous solvent containing a polyoxyethylene ester compound.

The term "aqueous solvent" in this specification is not particularly limited. Examples of the aqueous solvent include an embodiment of a solvent containing at least water. Examples also include an embodiment containing the following buffer and/or lower alcohol.

The polyoxyethylene ester compound is not particularly limited. Examples include sodium polyoxyethylene alkyl ether sulfate, polyoxyethylene alkyl ether phosphate, polyoxyethylene alkyl phenyl ether phosphate, poly(oxyethylene/oxypropylene)methylpolysiloxane copolymer, polyoxyethylene octyl phenyl ether, polyoxyethylene stearyl ether, polyoxyethylene stearic acid amide, polyoxyethylene cetyl ether, polyoxyethylene polyoxy, polyoxyethylene castor oil ester, and the like.

Among these, polyoxyethylene castor oil ester is preferable and polyoxy alkylene ($C_{24}$) castor oil fatty acid ester (Cremophor® EL) is more preferable.

By bringing lipid film (II) described above into contact with a polyoxyethylene ester compound, and then subjecting the matter obtained by the contact to a known liposome forming treatment, it is possible to produce a liposome encapsulating a poorly water-soluble pharmacologically active substance.

The method of the liposome forming treatment is not particularly limited. For example, a thin-film hydration method, ultrasonic treatment method, extruder treatment method, and the like may be used. Further, after the liposome forming treatment is performed, ultrafiltration using a membrane filter may be performed.

An embodiment of the aqueous solvent described above may also be a solvent containing lower alcohol in addition to water. The lower alcohol is not particularly limited. Examples include $C_{1-4}$ alcohols.

An embodiment of the aqueous solvent described above may further be a solvent containing a buffer in addition to water and/or lower alcohol. Examples of the buffer are not particularly limited, and include PBS, MES, ADA, PIPES, ACES, BES, TES, HEPES, and the like. Among these, PBS is preferable.

The amount of the polyoxyethylene ester compound in the aqueous solvent described above is not particularly limited. Generally, the amount is 10 to 30 parts by volume, preferably 15 to 25 parts by volume, further preferably 17 to 23 parts by volume, most preferably 18 to 22 parts by volume, per 100 parts by volume of the solvent.

"Parts by volume" is a value measured under an environment at atmospheric pressure and room temperature (about 15 to 40°C).

The measured value of the particle size of the liposome obtained by Method for producing a liposome encapsulating a poorly water-soluble pharmacologically active substance (III) of the present invention is not particularly limited. Generally, the particle size is about 200 nm or less.

Further, the measured value of zeta potential of the liposome obtained by Method for producing a liposome encapsulating a poorly water-soluble pharmacologically active substance (III) is also not particularly limited. Generally, the liposome may be an anionic liposome of about -10 mV.

An embodiment of Method for producing a liposome encapsulating a poorly water-soluble pharmacologically active substance (III) of the present invention may be a method further comprising the step of loading the liposome formed by the method above with an antibody recognizing a cancer cell. More specifically, the liposome encapsulating a poorly water-soluble pharmacologically active substance produced by the production method of the present invention may be a liposome carrying an antibody recognizing a cancer cell.

The antibody recognizing a cancer cell is not particularly limited. Examples of the antibody include immunoglobulin, antibody fragments such as Fab, and the like. Among these antibodies, immunoglobulin and IgG are preferable.

The method for loading the liposome with an antibody recognizing a cancer cell is not particularly limited. The loading may be performed by chemical modification using a linker.

The cancer cell is not particularly limited. Examples of cancer cells include lung cancer cells, non-small-cell lung cancer cells, breast cancer cells, esophageal cancer cells, gastric cancer cells, liver cancer cells, pancreatic cancer cells, colon cancer cells, ovarian cancer cells, cervical cancer cells, endometrial cancer cells, prostate cancer cells, head and neck cancer cells (including oral cancer cells, pharyngeal cancer cells, laryngeal cancer cells, nasal or nasal sinus cancer cells, salivary gland cancer cells, thyroid cancer cells), and the like.

Of these, based on the clinical application knowledge of paclitaxel, non-small-cell lung cancer cells, breast cancer cells, esophageal cancer cells, gastric cancer cells, endometrial cancer cells, ovarian cancer cells, prostate cancer cells, and the like, are preferable.

Examples of the antibody recognizing a cancer cell described above include an antibody specifically recognizing biomolecules such as proteins (e.g., CD proteins forming CD protein groups such as CD44 and CD133; receptors for growth factors or hormones; and proteins having a transmembrane domain or membrane-binding domain), peptides, sugar chains, lipids, and the like present on the surface layer of the cancer cell. The antibody is not particularly limited, and any known antibody expressed on the surface layer of each cancer cell may be appropriately selected.

Examples of the antibody recognizing a breast cancer cell include antibodies recognizing biomolecules such as proteins, peptides, sugar chains, lipids, and the like present on the surfaces of cells, for example, breast cancer cells extracted from breast cancer patients, more specifically, cells derived from breast cancer tissues, such as Hs274.T cell, Hs280.T cell, Hs281.T cell, Hs343.T cell, Hs362.T cell, Hs739.T cell, Hs741.T cell, Hs742.T cell, Hs190.T cell, Hs319.T cell, Hs329.T cell, Hs344.T cell, Hs350.T cell, Hs371.T cell, Hs748.T cell, Hs841.T cell, Hs849.T cell, Hs851.T cell, Hs861.T cell, Hs905.T cell, Hs479.T cell, Hs540.T cell, Hs566(B).T cell, Hs605.T cell, Hs606 cell, BT-20 cell, UACC-812 cell, HCC1954 cell, Hs574.T cell, BT-483 cell, BT-549 cell, DU4475 cell, Hs578T cell, BT-474 cell, UACC-893 cell, HCC38 cell, HCC70 cell, HCC202 cell, HCC1143 cell, HCC1187 cell, HCC1395 cell, HCC1419 cell, HCC1500 cell, HCC1599 cell, HCC1937 cell, HCC2157 cell, HCC2218 cell, HCC1569 cell, MB157 cell, SK-BR3 cell, MDA-MB-330 cell, MDA-MB-453 cell, MDA-MB-157 cell, MDA-MB-134 cell, T-47D cell, ZR-75 cell, and MCF-7 cell.

Specifically, examples of the antibody include anti-HER2 antibody (anti-ErbB2 antibody), anti-CEA antibody, and the like.

Examples of the antibody recognizing a lung cancer cell include antibodies recognizing biomolecules such as proteins, peptides, sugar chains, lipids, and the like present on the surfaces of cells, for example, lung cancer cells extracted from lung cancer patients, more specifically, cells derived from lung cancer tissues, such as Hs229.T cell, NCI-H2066 cell, NCI-H2286 cell, NCI-H1703 cell, Hs573.T cell, A549 cell, A427 cell, N417 cell, NCI-H596 cell, SW1573 cell, NCI-H835U cell, MC11 cell, NCI-H727 cell, NCI-H720 cell, NCI-H810 cell, NCI-H292 cell, NCI-H2126 cell, H69 cell, NCI-H1688 cell, NCI-H1417 cell, NCI-H1672 cell, NCI-H1836 cell, DMS79 cell, DMS53 cell, DMS114 cell, SW1271 cell, NCI-H2227 cell, NCI-H1963 cell, SHP-77 cell, H69 cell, H69AR cell, NCI-H2170 cell, NCI-H520 cell, and SW900 cell.

Specifically, examples of the antibody include anti-HER2 antibody, anti-EGFR antibody, anti-CEA antibody, and the like.

Examples of the antibody recognizing a non-small-cell lung cancer cell include antibodies recognizing biomolecules such as proteins, peptides, sugar chains, lipids, and the like present on the surfaces of cells, for example, non-small-cell lung cancer cells extracted from non-small-cell lung cancer patients, more specifically, cells derived from non-small-cell lung cancer tissues, such as NCI-H23 cell, NCI-H522 cell, NCI-H1435 cell, NCI-H1563 cell, NCI-H1651 cell, NCI-H1734 cell, NCI-H1793 cell, NCI-H1838 cell, NCI-H1975 cell, NCI-H2073 cell, NCI-H2085 cell, NCI-H2228 cell, NCI-H2342 cell, NCI-H2347 cell, NCI-H2135 cell, NCI-H2172 cell, and NCI-H2444 cell.

Specifically, examples of the antibody include anti-HER2 antibody, anti-EGFR antibody, and the like.

Examples of the antibody recognizing an esophageal cancer cell include antibodies recognizing biomolecules such as proteins, peptides, sugar chains, lipids, and the like present on the surfaces of cells, for example, esophageal cancer cells extracted from esophageal cancer patients, more specifically, cells derived from esophageal cancer tissues, such as SGF-3 cell, EC-YO cell, TE-1 cell, TE-2 cell, TE-3 cell, TE-4 cell, TE-5 cell, TE-6 cell, TE-7 cell, TE-8 cell, TE-9 cell, TE-10 cell, TE-11 cell, TE-12 cell, TE-13 cell, TE-14 cell, and TE-15 cell.

Specifically, examples of the antibody include anti-HER2 antibody, anti-EGFR antibody, and the like.

Examples of the antibody recognizing a gastric cancer cell include antibodies recognizing biomolecules such as proteins, peptides, sugar chains, lipids, and the like present on the surfaces of cells, for example, gastric cancer cells extracted from gastric cancer patients, more specifically, cells derived from gastric cancer tissues, such as AZ521 cell, AGS cell, SNU-1 cell, SNU-5 cell, SNU-16 cell, NCI-N87 cell, Hs746T cell, and KATO III cell.

Specifically, examples of the antibody include anti-HER2 antibody, anti-EGFR antibody, anti-CEA antibody, anti-SLX antibody, and the like.

Examples of the antibody recognizing a liver cancer cell include antibodies recognizing biomolecules such as proteins, peptides, sugar chains, lipids, and the like present on the surfaces of cells, for example, liver cancer cells extracted from liver cancer patients, more specifically, cells derived from liver cancer tissues, such as HepG2 cell, Huh-7 cell, C3A cell, SNU-398 cell, SNU-449 cell, SNU-182 cell, SNU-475 cell, Hep3B2.1-7 cell, PLHC-1 cell, SNU-387 cell, SNU-423 cell, and SK-HEP-1 cell.

Specifically, examples of the antibody include anti-HER2 antibody and the like.

Examples of the antibody recognizing a pancreatic cancer cell include antibodies recognizing biomolecules such as proteins, peptides, sugar chains, lipids, and the like present on the surfaces of cells, for example, pancreatic cancer cells extracted from pancreatic cancer patients, more specifically, cells derived from pancreatic cancer tissues, such as MIAPaCa-2 cell, BxPC-3 cell, HPAF-II cell, HPAC cell, Panc03.27 cell, Panc08.13 cell, Panc02.03 cell, Panc02.13 cell, Panc04.03 cell, Panc05.04 cell, Capan-2 cell, CFPAC-1 cell, PL45 cell, Panc10.05 cell, PANC-1 cell, AsPC-1 cell, Capan-1 cell, SW1990 cell, Hs766T cell, and SU.86.86 cell.

Specifically, examples of the antibody include anti-HER2 antibody, anti-CEA antibody, anti-SLX antibody, and the like.

Examples of the antibody recognizing a colon cancer cell include antibodies recognizing biomolecules such as proteins, peptides, sugar chains, lipids, and the like present on the surfaces of cells, for example, colon cancer cells extracted from colon cancer patients, more specifically, cells derived from colon cancer tissues, such as WiDr cell, Caco-2 cell, NCI-H548 cell, Hs255.T cell, TAC-1 cell, COLO320DM cell, COLO320HSR cell, DLD-1 cell, HCT-15 cell, SW480 cell, SW403 cell, SW48 cell, SW1116 cell, SW948 cell, SW1417 cell, LS123 cell, LS180 cell, LS174T cell, C2BBe1 cell, Hs257.T cell, Hs587.Int cell, HT-29 cell, HCT-8 cell, Hs675.T cell, HCT116 cell, ATRFLOX cell, Hs698.T cell, SW626 cell, SNU-C1 cell, COLO205 cell, COLO201 cell, SW620 cell, LoVo cell, SK-CO-1 cell, and T84 cell.

Specifically, examples of the antibody include anti-HER2 antibody, anti-EGFR antibody, anti-CEA antibody, and the like.

Examples of the antibody recognizing an ovarian cancer cell include antibodies recognizing biomolecules such as proteins, peptides, sugar chains, lipids, and the like present on the surfaces of cells, for example, ovarian cancer cells extracted from ovarian cancer patients, more specifically, cells derived from ovarian cancer tissues, such as PA-1 cell, Caov-3 cell, TOV-21G cell, TOV-112D cell, Hs38.T cell, Hs571.T cell, ES-2 cell, TE84.T cell, NIH:OVCAR-3 cell, SK-OV-3 cell, Caov-4 cell, and OV-90 cell.

Specifically, examples of the antibody include anti-HER2 antibody and the like.

Examples of the antibody recognizing a cervical cancer cell include antibodies recognizing biomolecules such as proteins, peptides, sugar chains, lipids, and the like present on the surfaces of cells, for example, cervical cancer cells extracted from cervical cancer patients, more specifically, cells derived from cervical cancer tissues, such as HeLa cell, HeLa229 cell, HeLaS3 cell, H1HeLa cell, Hs588.T cell, GH329 cell, GH354 cell, HeLaNR1 cell, C-4I cell, C-4II cell, DoTc2 4510 cell, C-33A cell, SW756 cell, SiHa cell, HT-3 cell, MS751 cell, CaSki cell, and ME-180 cell.

Specifically, examples of the antibody include anti-HER2 antibody and the like.

Examples of the antibody recognizing an endometrial cancer cell include antibodies recognizing biomolecules such as proteins, peptides, sugar chains, lipids, and the like present on the surfaces of cells, for example, endometrial cancer cells extracted from endometrial cancer patients, more specifically, cells derived from endometrial cancer tissues, such as HHUA cell, KLE cell, HEC-1-A cell, HEC-1-B cell, HEC-6 cell, HEC-50 cell, HEC-59 cell, HEC-108 cell, HEC-116 cell, RL95-2 cell, SK-UT-1 cell, SK-UT-1B cell, MES-SA cell, MES-SA/Dx5 cell, MES-SA/MX2 cell, AN3CA cell, SNG-P cell, and SNG-M cell.

Specifically, examples of the antibody include anti-HER2 antibody, anti-CEA antibody, and the like.

Examples of the antibody recognizing a prostate cancer cell include antibodies recognizing biomolecules such as proteins, peptides, sugar chains, lipids, and the like present on the surfaces of cells, for example, prostate cancer cells extracted from prostate cancer patients, more specifically, cells derived from prostate cancer tissues, such as LNCaP cell, 22Rv1 cell, PC-3 cell, MDA PCa 2b cell, TRAMP-C3 cell, DU145 cell, NCI-H660 cell, TSU-PR1PC-82 cell, PPC-1 cell, and VCRU-Pr-2 cell.

Specifically, examples of the antibody include anti-HER2 antibody, anti-EGFR antibody, and the like.

Examples of the antibody recognizing an oral cancer cell, which is a head and neck cancer cell, include antibodies recognizing biomolecules such as proteins, peptides, sugar chains, lipids, and the like present on the surfaces of cells, for example, oral cancer cells extracted from oral cancer patients, more specifically, cells derived from oral cancer tissues, such as Hs53.T cell.

Examples of the antibody recognizing a pharyngeal cancer cell, which is a head and neck cancer cell, include antibodies recognizing biomolecules such as proteins, peptides, sugar chains, lipids, and the like present on the surfaces of cells, for example, pharyngeal cancer cells extracted from pharyngeal cancer patients, more specifically, cells derived from pharyngeal cancer tissues, such as C666-1 cell, NPC-TY861 cell, MPC-Y851 cell, MPC-K852 cell, KKK-YT cell, and MPC-ST cell.

Examples of the antibody recognizing a laryngeal cancer cell, which is a head and neck cancer cell, include antibodies recognizing biomolecules such as proteins, peptides, sugar chains, lipids, and the like present on the surfaces of cells, for example, laryngeal cancer cells extracted from laryngeal cancer patients, more specifically, cells derived from laryngeal cancer tissues, such as FaDu cell, Hs840.T cell, and Detroit 562 cell.

Examples of the antibody recognizing a nasal or nasal sinus cancer cell, which is a head and neck cancer cell, include antibodies recognizing biomolecules such as proteins, peptides, sugar chains, lipids, and the like present on the surfaces of cells, for example, nasal or nasal sinus cancer cells extracted from nasal or nasal sinus cancer patients, more specifically, cells derived from nasal or nasal sinus cancer tissues, such as RPMI2650 cell.

Examples of the antibody recognizing a salivary gland cancer cell, which is a head and neck cancer cell, include antibodies recognizing biomolecules such as proteins, peptides, sugar chains, lipids, and the like present on the surfaces of cells, for example, salivary gland cancer cells extracted from salivary gland cancer patients, more specifically, cells derived from salivary gland cancer tissues, such as SGT-1 cell.

Examples of the antibody recognizing a thyroid cancer cell, which is a head and neck cancer cell, include antibodies recognizing biomolecules such as proteins, peptides, sugar chains, lipids, and the like present on the surfaces of cells, for example, thyroid cancer cells extracted from thyroid cancer patients, more specifically, cells derived from thyroid cancer tissues, such as HTC/C3 cell, SW579 cell, and TT cell.

Specifically, examples of these antibodies recognizing head and neck cancers include anti-HER2 antibody, anti-EGFR antibody, and the like.

The method for loading such a liposome of the present invention with an antibody recognizing a cancer cell is not particularly limited. For example, the method disclosed in PTD 1 may be used.

## IV. Liposome Formulation

The liposome formulation of the present invention comprises a liposome obtained by Method for producing a liposome encapsulating a poorly water-soluble pharmacologically active substance (III) described above.

More specifically, the liposome formulation of the present invention is a liposome formulation comprising a liposome containing a lipid having a phosphatidylcholine group, a cholesterol compound, a lipid having a phosphatidylethanolamine group, a poorly water-soluble pharmacologically active substance, a polyoxyethylene ester compound, as well as a pharmaceutically acceptable carrier and additives, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

The liposome formulation may be used for the prevention or treatment of diseases based on the various diseases in which the poorly water-soluble pharmacologically active substance encapsulated in the liposome exhibits the therapeutic effects.

For example, when a taxane-based compound typified by paclitaxel, docetaxel, cabazitaxel, and glycosides thereof is used as the poorly water-soluble pharmacologically active substance to be encapsulated in the liposome, the liposome formulation comprising the liposome may be applied to the prevention or treatment of cancers, Alzheimer's, atopic dermatitis, ulcerative colitis, rheumatoid arthritis (autoimmune diseases), gastritis caused by *Helicobacter pylori,* viral hepatitis (infectious inflammation), and the like. The formulation may also be used for a purpose that exhibits, for example, an antipyretic, analgesic, antitussive, bacterial, immunosuppressive, or antiparasitic effect.

For example, when a macrolide compound typified by bafilomycin, bafilomycin, concanamycin, azithromycin, and clarithromycin is used as the poorly water-soluble pharmacologically active substance to be encapsulated in the liposome formulation, the liposome formulation comprising the liposome may be applied to the prevention or treatment of cancers, Alzheimer's, atopic dermatitis, ulcerative colitis, rheumatoid arthritis (autoimmune diseases), gastritis caused by *Helicobacter pylori,* viral hepatitis (infectious inflammation), and the like. The formulation may also be used for the purpose that exhibits, for example, an antipyretic, analgesic, antitussive, bacterial, immunosuppressive, or antiparasitic effect.

For example, when a quinoline alkaloid compound typified by camptothecin and irinotecan is used as the poorly water-soluble pharmacologically active substance to be encapsulated in the liposome formulation, the liposome formulation comprising the liposome may be applied to the prevention or treatment of cancers, Alzheimer's, atopic dermatitis, ulcerative colitis, rheumatoid arthritis (autoimmune diseases), gastritis caused by *Helicobacter pylori,* viral hepatitis (infectious inflammation), and the like. The formulation may also be used for the purpose that exhibits, for example, an antipyretic, analgesic, antitussive, bacterial, immunosuppressive, or antiparasitic effect.

For example, when a vinca alkaloid compound typified by vincristine, vinblastine, vindesine, and vinorelbine is used as the poorly water-soluble pharmacologically active substance to be encapsulated in the liposome formulation, the liposome formulation comprising the liposome may be applied to the prevention or treatment of cancers, Alzheimer's, atopic dermatitis, ulcerative colitis, rheumatoid arthritis (autoimmune diseases), gastritis caused by *Helicobacter pylori,* viral hepatitis (infectious inflammation), and the like. The formulation may also be used for the purpose that exhibits, for example, an antipyretic, analgesic, antitussive, bacterial, immunosuppressive, or antiparasitic effect.

The cancers are not particularly limited. Examples of cancer include lung cancer cells, non-small-cell lung cancer, breast cancer cells, esophageal cancer, gastric cancer cell, liver cancer cells, pancreatic cancer cells, colon cancer cells, ovarian cancer, cervical cancer cells, endometrial cancer cells, prostate cancer cells, head and neck cancer cells (oral cancer cells, pharyngeal cancer cells, laryngeal cancer cells, nasal or nasal sinus cancer cells, salivary gland cancer cells, thyroid cancer cells, and the like).

The liposome formulation of the present invention may be administered, for example, to patients with the various diseases listed above. The administration route is not particularly limited. Examples of the administration include intravenous injection such as drip infusion, intramuscular injection, intraperitoneal injection, subcutaneous injection and the like. An appropriate administration method may be selected according to the age and the symptoms of the patient.

As a specific administration method of the liposome formulation, a pharmaceutical composition may be administered using a syringe or drip infusion. Further, it is also possible to insert a catheter into the patient's body, for example, into the lumen or blood vessel, and lead the catheter tip to the vicinity of the target site, and then administer the formulation from the desired target site, the vicinity thereof, or the site expected to have a bloodstream toward the target site, via the catheter.

The liposome formulation of the present invention is administered to patients with the various diseases listed above in an amount sufficient to treat or at least partially inhibit the symptoms of the diseases.

The effective dose of the drug encapsulated in the liposome formulation is not particularly limited. For example, the dose may be in a range of about 0.01 to 50 mg/kg when converted to the amount of the poorly water-soluble pharmacologically active substance encapsulated in the liposome formulation.

The liposome formulation of the present invention may contain a pharmaceutically acceptable carrier and additives. The pharmaceutically acceptable carrier and the additives are not particularly limited. Various known carriers and additives that have been used in this field may be used.

Examples are shown below to more specifically describe the present invention. However, the present invention is not limited to these Examples.

Production Example 1

Liposome Encapsulating PTX, gPTX, or DTX

A liposome encapsulating PTX (paclitaxel), gPTX (7-glucosyloxyacetylpaclitaxel), or DTX (docetaxel) was prepared by the thin-film hydration method.

9.6 mg of hydrogenated soy lecithin (HSPC), 3.2 mg of cholesterol (Chol), 3.2 mg of 1,2-distealoyl-sn-glycerol-3-phosphoethanolamine-N-[methoxy(polyethylene glycol)-2000](mPEG-DSPE) and a taxane compound (PTX, gPTX, or DTX) were weighed and added to an eggplant flask at a molar ratio of HSPC:Chol:mPEG-DSPE:taxane compound = 6:4:0.5:X.

Specifically, 0.9, 1.8, or 3.5 mg (x=0.5 to 2) of PTX, 2.2, 4.4, or 6.6 mg (x=1 to 3) of gPTX, and 1.7, 3.3 or 5.0 mg (x=1 to 3) of DTX were weighed and added to an eggplant flask.

4 mL of organic solvent (chloroform:methanol = 9:1) was added to the eggplant flask, and the mixed lipids were dissolved well. Thereafter, mixed lipids were vacuum-dried using a rotary evaporator to completely remove the organic solvent, and a lipid film encapsulating a taxane compound was formed.

1 mL of CEP (Cremophor EL:ethanol:phosphate buffered saline (PBS)) = 20:15:65 (volume ratio)) was added to the lipid film thus produced, and suspended while heating to 60°C, thereby forming a liposome. To adjust the particle size, a sonication treatment was performed under heating at 60°C. Thereafter, in order to remove any unencapsulated drug by ultrafiltration using a 100-KDa membrane filter, the liposome external liquid was replaced with PBS.

The particle size and zeta potential of the liposome thus prepared were measured by dynamic light scattering method and electrophoretic light scattering method using an ELS-8000 (Otsuka Electronics Co., Ltd.). Further, the drug concentration in the liposome was measured by reverse-phase HPLC, and the encapsulation efficiency and the loading efficiency were calculated.

The concentration of the drug encapsulated in the liposome was determined by reverse-phase high-performance liquid chromatography (reverse-phase HPLC). The measurement conditions were as follows. A WP300, C18.5 $\mu$m, 4.6×150 mm, was used as an HPLC column. A detection wavelength of 227 nm was used for PTX and gPTX, and a detection wavelength of 229 nm was used for DTX. A solvent of methanol:ultrapure water = 7:3 was used as a mobile phase. More specifically, 10 $\mu$L of a liposome sample was injected into an HPLC column, and the fluid was delivered in a mobile phase at a flow rate of 1.0 mL/min.

Using the following formulae (1) and (2), the encapsulation efficiency and the loading efficiency were calculated based on the obtained drug amount.

$$\text{Encapsulation efficiency (EE:\%) = drug amount/amount of drug}$$
$$\text{initially used} \times 100 \quad (1)$$

$$\text{Loading efficiency (LE:\%) = \{(drug amount/drug mol}$$
$$\text{weight)/initial lipid mol number\}} \times 100 \quad (2)$$

Production Example 2

Liposome Encapsulating Bafilomycin A1 (BafA1)

A liposome encapsulating BafA1 was prepared by the thin-film hydration method. 9.6 mg of HSPC, 3.2 mg of Chol, 3.2 mg of mPEG-DSPE and BafA1 were weighed and added to an eggplant flask at a molar ratio of HSPC:Chol:mPEG-DSPE=6:4:0.5:x.
145 $\mu$g or 290 $\mu$g (x=0.1 or 0.2) of BafA1 was weighed and added thereto.
4 mL of organic solvent (chloroform:methanol = 9:1) was added to the eggplant flask, and the mixed lipids were dissolved well. Thereafter, mixed lipids were vacuum-dried using a rotary evaporator to completely remove the organic solvent, and a lipid film encapsulating BafA1 was formed.
The liposome formation treatment using the lipid film encapsulating BafA1 thus produced, and the property evaluation of the liposome were performed in the same manner as in the preparation of the liposome encapsulating a taxane compound.
The concentration of BafA1 encapsulated in the liposome was determined by reverse-phase HPLC with the following measurement conditions. A WP300, C18.5 $\mu$m, 4.6$\times$150 mm, was used as an HPLC column. A detection wavelength of 245 nm was selected, and a solvent of methanol:ultrapure water = 8:3 was used as a mobile phase. More specifically, 10 $\mu$L of a liposome sample was injected into a HPLC column, and the fluid was delivered in a mobile phase at a flow rate of 1.0 mL/min.
Using formulae (1) and (2) shown above, the encapsulation efficiency and the loading efficiency were calculated.

Example 1

Liposome Stability Test at 4°C

After the liposome encapsulating a taxane compound described above was prepared, the liposome was left still at 4°C. After 2 weeks and after 4 weeks, any drug leaked into the liposome external liquid was removed by ultrafiltration using a 100-KDa membrane filter. Thereafter, the particle size of the liposome was measured by dynamic light scattering method; further, the drug concentration in the liposome solution was determined by reverse-phase HPLC. Using the following formula (3), the retention rate was calculated based on the obtained drug amount. Retention rate (%) = encapsulated drug amount/amount of drug initially encapsulated $\times$ 100 (3)

Example 2

Cytotoxicity Evaluation

Cytotoxicity of the liposome encapsulating PTX, gPTX, or DTX was evaluated by using an MTT assay. As the test target cells, HT-29 cell, which is a cell line derived from human colon cancer, SK-OV-3 cell, which is a cell line derived from human ovarian cancer, and SK-BR-3, which is a cell line derived from human breast cancer, were used. The cancer cells were seeded to a 96-well plate at 5000 cells/well.
After 24-hour culture, drugs with various concentrations were added to each well. After exposure to the drug for 72 hours, an MTT solution was added at a final concentration of 0.5 mg/mL, followed by culture for 4 hours. Thereafter, the generated formazan was dissolved in a formazan solution liquid (10% SDS+0.02-regulated HCl). The concentration ($IC_{50}$) at which 50% of the cells die was calculated from the 470-nm cell survival curve of each well.
Cytotoxicity of BafA1 and the liposome encapsulating BafA1 was evaluated by using the following MTT assay. As test target cells, HT-29 cell, which is a cell line derived from human colon cancer, and MCF7, which is a cell line derived from human breast cancer, were used. The cancer cells were seeded to a 96-well plate at 5000 cells/well. After 24-hour culture, drugs with various concentrations were added to each well. After exposure to the drug for 48 hours, an MTT solution was added at a final concentration of 0.8 mg/mL, followed by culture for 2 hours. Thereafter, the generated formazan was dissolved in a formazan solution liquid (10% SDS+0.02-regulated HCl). The concentration ($IC_{50}$) at which

50% of the cells die was calculated from the 470-nm cell survival curve of each well.

Comparative Example 1

Comparison with Preparation of a Liposome Encapsulating Paclitaxel Using a Known Lipid Constitution (NPL1)

9.6 mg of HSPC, 3.2 mg of Chol, 3.2 mg of mPEG-DSPE, and 1.8 mg of PTX, or 14.5 mg of HSPC, 0.8 mg of Chol, 2.8 mg of mPEG-DSPE, and 1.8 mg of PTX were weighed (9:1:0.5:1 (molar ratio)) and added to an eggplant flask. 4 mL of organic solvent (chloroform:methanol = 9:1) was added to the eggplant flask, and the mixed lipids were dissolved well. Thereafter, the lipid solution was vacuum-dried using a rotary evaporator to completely remove the organic solvent, and a lipid film encapsulating an anticancer drug was formed.

1 mL of CEP (Cremophor EL:ethanol:phosphate buffered saline (PBS) = 20:15:65 (volume ratio)) was added to the formed lipid film, followed by suspension while heating to 60°C, thereby forming a liposome. The formation of the obtained multilayer membrane liposome was observed with a microscope.

Results of Various Tests

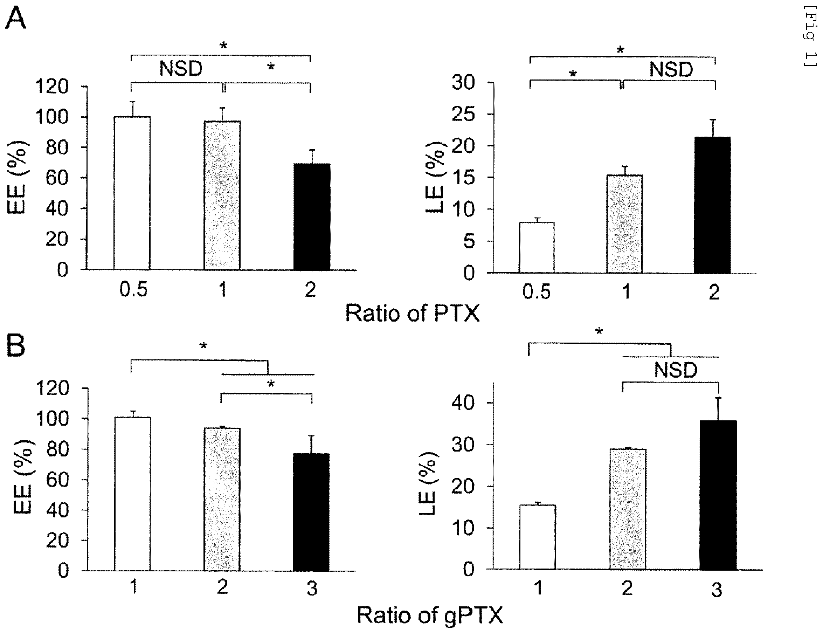

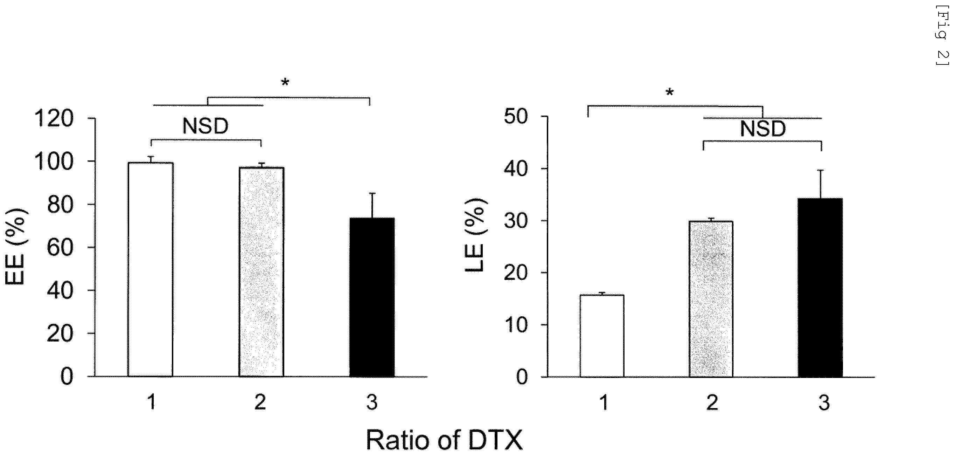

Figs. 1 and 2 show the results of measuring encapsulation efficiency (EE:%) and loading efficiency (LE:%) of the respective taxane compounds, i.e., PTX, gPTX, and DTX, in the liposome.

The results confirmed that the encapsulation efficiency was nearly 100% when the molar ratio was 5 mol and 10 mol for PTX, when the molar ratio was 10 mol for gPTX, and when the molar ratio was 10 mol and 20 mol for DTX. In each drug, there was a tendency for the loading efficiency to increase as the molar ratio increases.

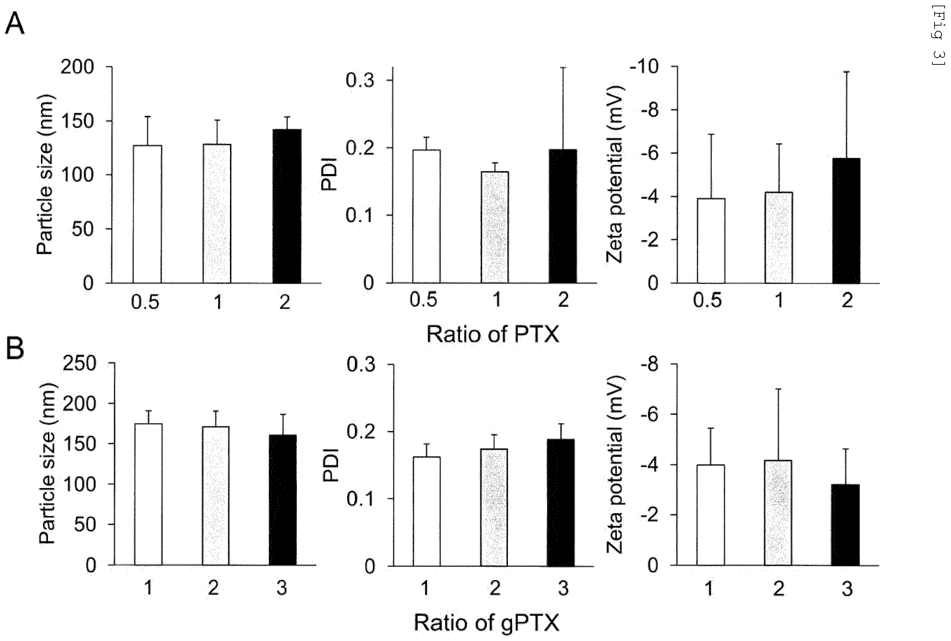

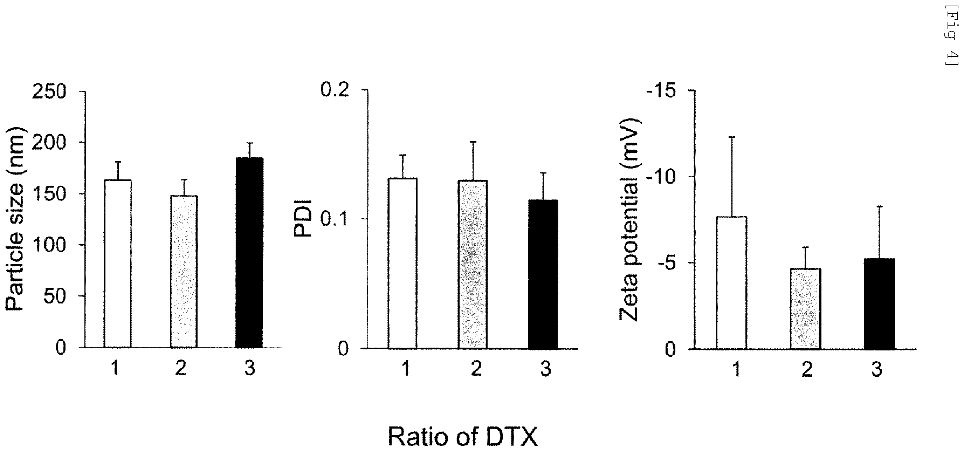

The property evaluation of the liposomes encapsulating taxane compounds shown in Figs. 3 and 4 suggested that all of the liposomes are usable as a liposome formulation in terms of particle size, polydispersity index, and zeta potential. In particular, the particle size of 200 nm or less is suitable to ensure EPR effects, and the negative zeta potential is desirable in terms of preventing easy recognition in the liver.

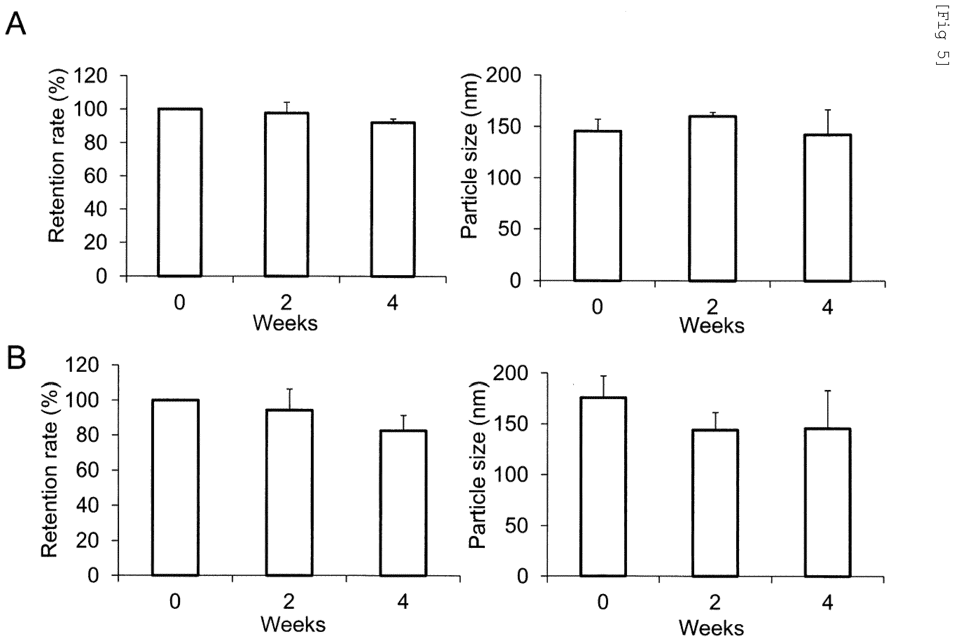

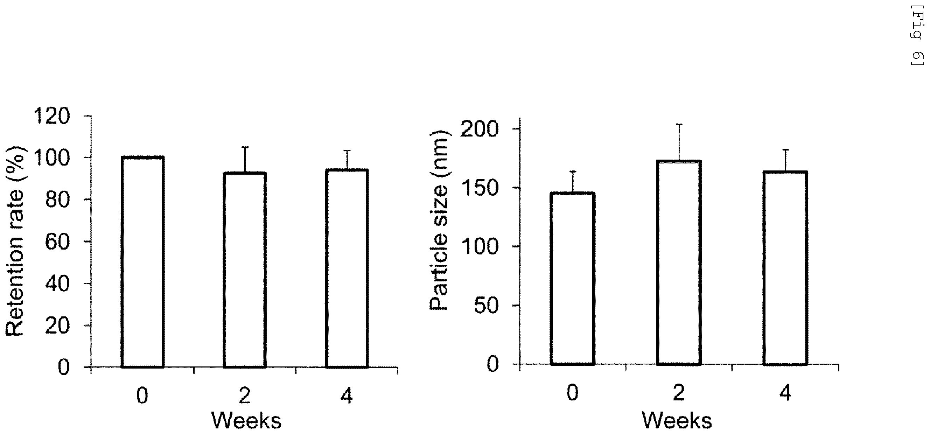

According to the retention rates shown in Figs. 5 and 6, it was suggested that all of the liposomes encapsulating taxane compounds were useful as a liposome formulation. Further, no significant change in particle size was confirmed during the 4-week preservation period at 4°C.

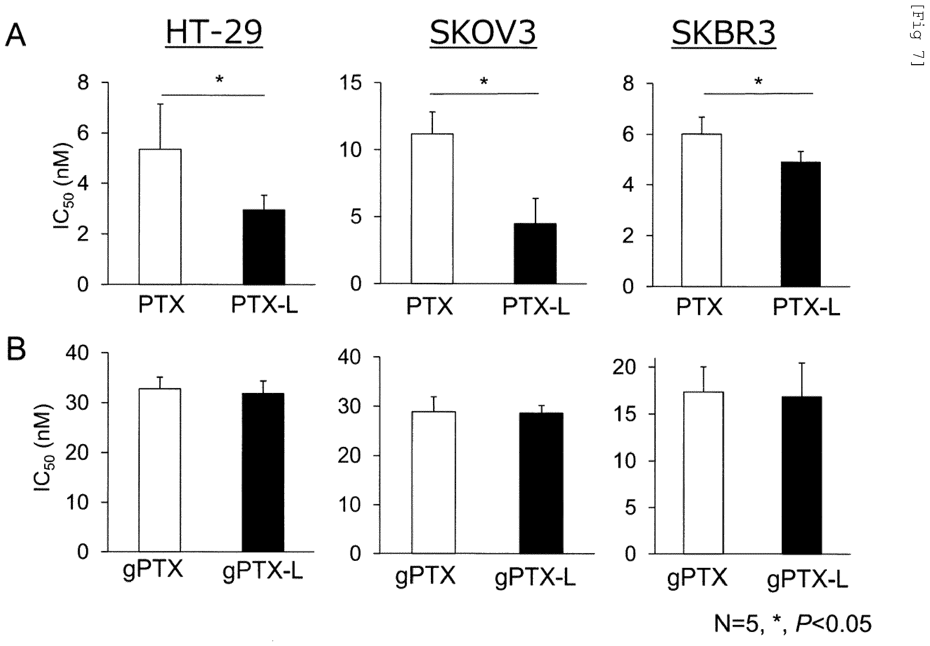

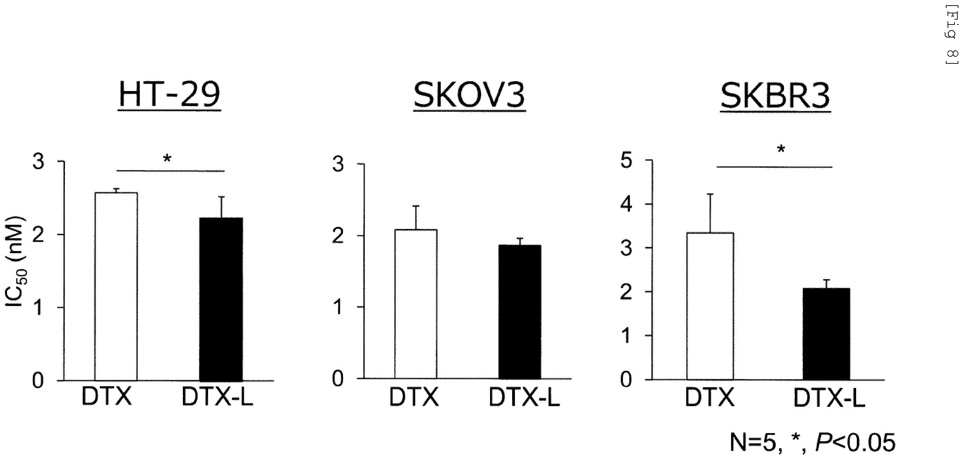

Figs. 7 and 8 show the evaluation of anti-cancer activity of the respective liposomes encapsulating taxane compounds, i.e., PTX, gPTX, and DTX, in a cancer cell. The results revealed that all liposomes encapsulating various taxane compounds exhibited preferable anti-cancer activity against various cancer cells. In particular, it was revealed that PTX exhibited more desirable anti-cancer activity when it was encapsulated in a liposome.

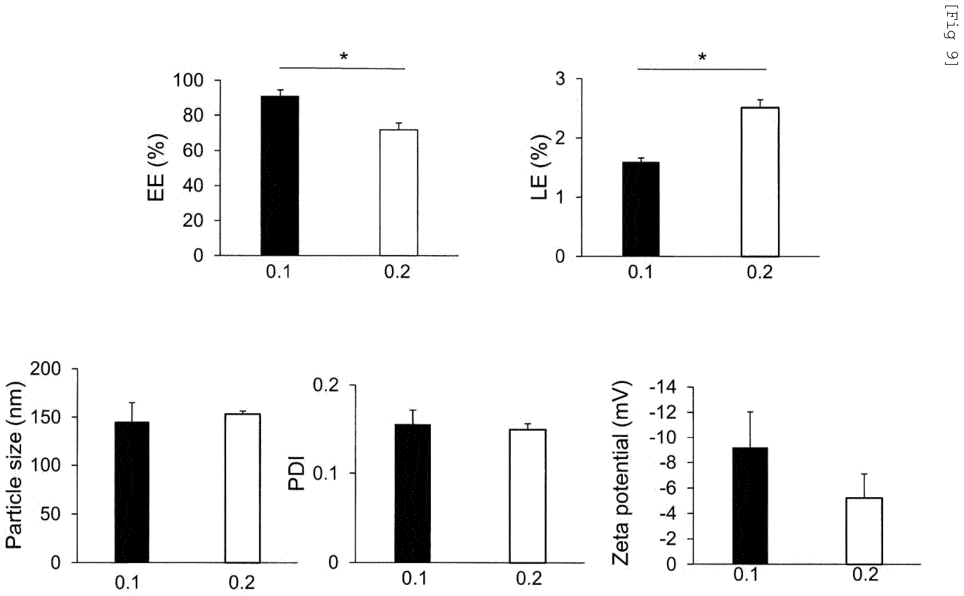

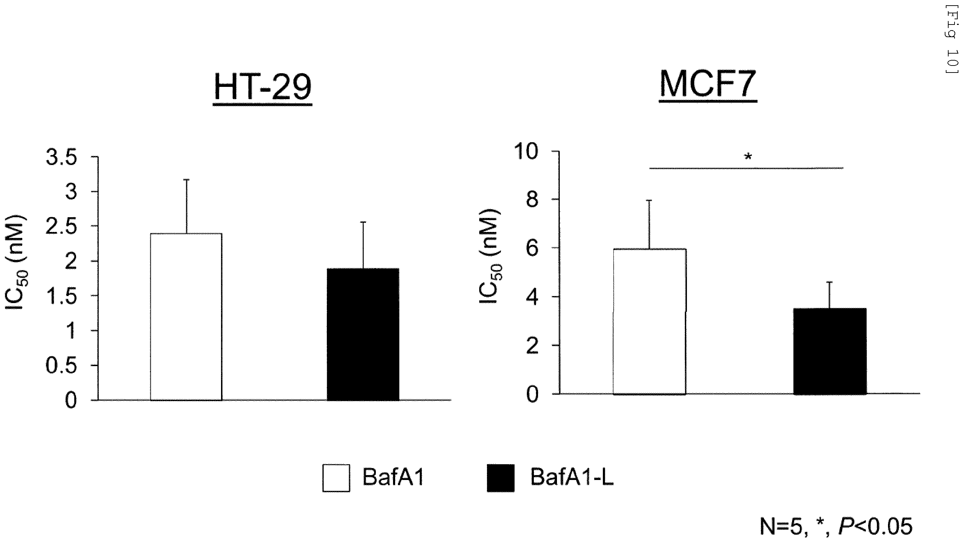

Figs. 9 and 10 show test results with respect to the liposomes encapsulating bafilomycin; the test was performed in the same manner as in the test for various taxane compounds. The encapsulation efficiency (EE:%) of bafilomycin into a liposome shown in Fig. 9 revealed that nearly 100% of encapsulation efficiency was achieved when the molar ratio was 1 mol, as in the taxane compound. Also, there was a tendency for the loading efficiency (LE:%) to increase as the molar ratio increases. Further, with regard to the particle size and zeta potential, the same tendency as that of the taxane-based drug was confirmed.

Further, the results of Fig. 10 confirmed that BafA1 encapsulated in a liposome exhibited cytotoxicity to the same as or greater extent than that of BafA1.

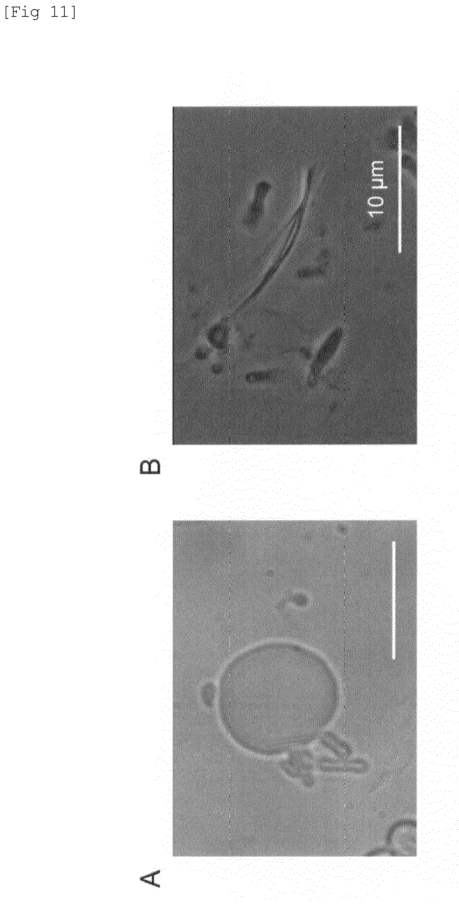

The results of Fig. 11 revealed that when a lipid film encapsulating paclitaxel at a known lipid constitution was produced and processed into a liposome (see Fig. 11(B)), a liposome membrane like the one shown in Fig. 11(A) was not formed. The structure of such a needle-like form was not clarified; however, it is considered to be a paclitaxel aggregation.

Example 3

Liposome Survival Test

The liposome described above was subjected to a survival test to confirm the safety as a liposome formulation.

SPF/VAF mice (strain: BALB/cAnNCr1Cr1j; Charles River Laboratories Japan, Inc.) were used. Six-week-old female BALB/c mice were classified into groups each having 4 mice. The mice were raised in an environment at 23°C and fed with sterilized water and food.

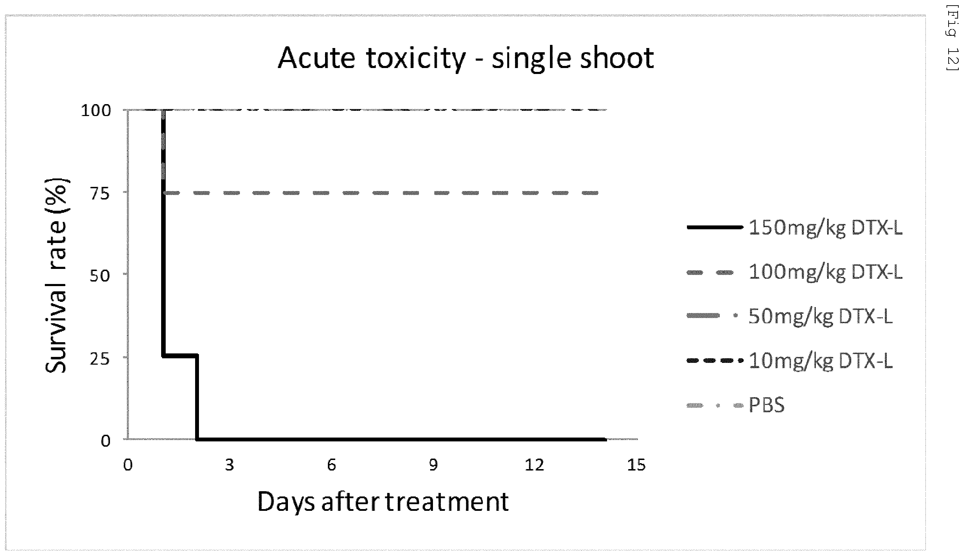

Among the docetaxel liposomes (DTX-L) dissolved in a physiological saline, the DTX-L obtained by using docetaxel in a molar amount (x) of 2 relative to the entire liposome was administered to each mouse via tail vein in an amount of 10, 50, 100, or 150 mg/kg in terms of the docetaxel amount. Further, PBS was administered in a similar manner as a control. Fig. 12 shows the results.

When DTX-L in an amount of 150 mg/kg in terms of docetaxel amount was added, the survival rate 1 day after the administration was 25%, and was 0% two days after the administration. When DTX-L in an amount of 100 mg/kg in terms of docetaxel amount was added, the survival rate 1 day after the administration decreased to 75%, and no change was observed thereafter until 15 days after the administration.

Therefore, it was suggested that the upper limit of the administration amount of the DTX-L was about 50 mg/kg. Since the DTX-L encapsulates docetaxel at an encapsulation efficiency of at least about 95%, administration of about 3 g or more is possible for a patient weighing 66 kg according to the calculation based on the upper limit. This value is clearly much larger than the currently approved docetaxel dosage value.

**Claims**

1. A composition comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

2. The composition according to claim 1, wherein the lipid having a phosphatidylcholine group is at least one member selected from the group consisting of hydrogenated soy lecithin, egg yolk phospholipid, distearoyl phosphatidylcholine, dimyristoyl phosphatidylcholine, dipalmitoyl phosphatidylcholine, and dioleoyl phosphatidylcholine.

3. The composition according to claim 1 or 2, wherein the cholesterol compound is at least one member selected from the group consisting of cholesterol, cholestanol, 7-dehydrocholesterol, and phytosterol.

4. The composition according to any one of claims 1 to 3, wherein the lipid having a phosphatidylethanolamine group is at least one member selected from the group consisting of distearoyl phosphatidylethanolamine, dipalmitoyl phosphatidylethanolamine, dimyristoyl phosphatidylethanolamine, and dioleoyl phosphatidylethanolamine.

5. The composition according to any one of claims 1 to 4, wherein any one of the lipid having a phosphatidylethanolamine group, the cholesterol compound, and the lipid having a phosphatidylcholine group is modified by polyalkylene glycol.

6. The composition according to any one of claims 1 to 5, wherein the poorly water-soluble pharmacologically active substance is at least one member selected from the group consisting of taxane compound, macrolide compound, vinca alkaloid compound, quinoline alkaloid compound, and etoposide compound.

7. The composition according to claim 6, wherein the taxane compound is at least one member selected from the group consisting of paclitaxel, docetaxel, cabazitaxel, and glycosides thereof.

8. The composition according to claim 6, wherein the macrolide compound is at least one member selected from the group consisting of bafilomycin, concanamycin, azithromycin, and clarithromycin.

9. The composition according to any one of claims 1 to 8, wherein the composition is used to form a lipid film.

10. A lipid film comprising a lipid having a phosphatidylcholine group, a cholesterol compound, a lipid having a phosphatidylethanolamine group, and a poorly water-soluble pharmacologically active substance, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

11. A method for producing a liposome encapsulating a poorly water-soluble pharmacologically active substance, comprising the step of bringing the lipid film of claim 10 into contact with a polyoxyethylene ester compound in an aqueous solvent.

12. The method according to claim 11, wherein lower alcohol is further contained as an aqueous solvent.

13. The method according to claim 11 or 12, wherein a buffer is further contained as an aqueous solvent.

**14.** The method according to any one of claims 11 to 13, further comprising the step of loading an antibody recognizing a cancer cell.

**15.** A liposome formulation comprising a liposome encapsulating a lipid having a phosphatidylcholine group, a cholesterol compound, a lipid having a phosphatidylethanolamine group, a poorly water-soluble pharmacologically active substance, and a polyoxyethylene ester compound, wherein the molar ratio of the lipid having a phosphatidylcholine group, the cholesterol compound, the lipid having a phosphatidylethanolamine group, and the poorly water-soluble pharmacologically active substance is 3 to 8:2 to 7:0.1 to 3:0.001 to 5, respectively.

[Fig 1]

EP 3 360 577 A1

[Fig 2]

EP 3 360 577 A1

[Fig 3]

[Fig 4]

EP 3 360 577 A1

Ratio of DTX

[Fig 5]

[Fig 6]

[Fig 7]

EP 3 360 577 A1

A    HT-29    SKOV3    SKBR3

B

N=5, *, *P*<0.05

[Fig 8]

[Fig 9]

[Fig 10]

[Fig 11]

[Fig 12]

EP 3 360 577 A1

Acute toxicity - single shoot

150mg/kg DTX-L

100mg/kg DTX-L

50mg/kg DTX-L

10mg/kg DTX-L

PBS

# EP 3 360 577 A1

<table>
<tr><td align="center">INTERNATIONAL SEARCH REPORT</td><td>International application No.<br>PCT/JP2016/079837</td></tr>
</table>

**A. CLASSIFICATION OF SUBJECT MATTER**

*A61K47/24*(2006.01)i, *A61K9/127*(2006.01)i, *A61K31/337*(2006.01)i, *A61K31/357* (2006.01)i, *A61K31/4545*(2006.01)i, *A61K31/7048*(2006.01)i, *A61K39/395* (2006.01)i, *A61K45/00*(2006.01)i, *A61K47/10*(2006.01)i, *A61K47/28*(2006.01)i,

According to International Patent Classification (IPC) or to both national classification and IPC

**B. FIELDS SEARCHED**

Minimum documentation searched (classification system followed by classification symbols)
A61K47/24, A61K9/127, A61K31/337, A61K31/357, A61K31/4545, A61K31/7048, A61K39/395, A61K45/00, A61K47/10, A61K47/28, A61K47/34, A61P35/00

Documentation searched other than minimum documentation to the extent that such documents are included in the fields searched
Jitsuyo Shinan Koho 1922–1996 Jitsuyo Shinan Toroku Koho 1996–2016
Kokai Jitsuyo Shinan Koho 1971–2016 Toroku Jitsuyo Shinan Koho 1994–2016

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2009-541319 A (The Johns Hopkins University),<br>26 November 2009 (26.11.2009),<br>paragraphs [0034] to [0036]<br>& US 2011/0250258 A1<br>paragraphs [0038] to [0041]<br>& US 2013/0142862 A1 & US 2013/0315986 A1<br>& US 2015/0306184 A1 & WO 2007/149433 A2<br>& KR 10-2009-0027674 A & CN 101489593 A | 1-6,9,10,15<br>8,11-14 |
| X<br><br>Y | WO 2007/089043 A1 (Takeda Chemical Industries, Ltd.),<br>09 August 2007 (09.08.2007),<br>example 1<br>& US 2009/0169610 A1<br>example 1<br>& EP 1980243 A1 & CA 2640598 A | 1-4,6,7,9,<br>10,15<br>8,11-14 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>05 December 2016 (05.12.16) | Date of mailing of the international search report<br>13 December 2016 (13.12.16) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

30

**INTERNATIONAL SEARCH REPORT**

International application No.

PCT/JP2016/079837

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2012-236772 A (Terumo Corp.),<br>06 December 2012 (06.12.2012),<br>tables 1, 3, 5, 6<br>& WO 2011/037252 A1 | 1-5,9,10,15<br>8 |
| X<br>Y | JP 2006-508126 A (Azaya Therapeutics, Inc.),<br>09 March 2006 (09.03.2006),<br>paragraph [0068]<br>& US 2004/0247660 A1<br>paragraph [0076]<br>& US 2007/0166368 A1    & WO 2004/043363 A2<br>& EP 1585504 A2          & CA 2505520 A<br>& AU 2003287526 A | 1-7,15<br>8 |
| X<br>Y | WO 2012/035794 A1 (Nanosion Co., Ltd.),<br>22 March 2012 (22.03.2012),<br>example 1<br>& US 2013/0171243 A1<br>example 1<br>& AU 2011303262 A        & CA 2811494 A<br>& SG 188554 A            & KR 10-2013-0108583 A<br>& IL 225288 D | 1-6,15<br>8 |
| X<br>Y | JP 2002-527466 A (Alza Corp.),<br>27 August 2002 (27.08.2002),<br>paragraph [0045]<br>& US 6355268 B1          & US 2002/0146450 A1<br>paragraphs [0095], [0096]<br>& WO 2000/023052 A1      & EP 1121102 A1<br>& DE 69907243 D          & DE 69907243 T<br>& AU 1118900 A           & NO 20011844 A<br>& BR 9914601 A           & CA 2346879 A<br>& IL 142573 D            & AT 238039 T | 1-6,15<br>8 |
| Y | WO 2012/102362 A1 (Kowa Co., Ltd.),<br>02 August 2012 (02.08.2012),<br>paragraph [0014]<br>& JP 5906195 B2          & CN 103338789 A<br>& KR 10-2014-0005930 A  & RU 2013139558 A<br>& TW 201309344 A | 8 |
| Y | WO 2013/141346 A1 (Ensuiko Sugar Refining Co.,<br>Ltd.),<br>26 September 2013 (26.09.2013),<br>test example 6<br>& US 2015/0056270 A1<br>test example 6<br>& EP 2829273 A1          & CN 104203251 A | 11-14 |
| Y | TAO Yang, et al., Enhanced solubility of<br>PEGylated liposomal paclitaxel: In vitro and in<br>vivo evaluation, International Journal of<br>Pharmaceutics, 2007, vol.338, 317-326, page 318,<br>right column, 2nd paragraph | 11-14 |

Form PCT/ISA/210 (continuation of second sheet) (January 2015)

**INTERNATIONAL SEARCH REPORT**

International application No.

PCT/JP2016/079837

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2016-160248 A (Kyoto University), 05 September 2016 (05.09.2016), paragraphs [0078] to [0080] (Family: none) | 1-5,9,10,15 |

Form PCT/ISA/210 (continuation of second sheet) (January 2015)

**INTERNATIONAL SEARCH REPORT**

International application No.

PCT/JP2016/079837

Continuation of A. CLASSIFICATION OF SUBJECT MATTER
 (International Patent Classification (IPC))

*A61K47/34*(2006.01)i, *A61P35/00*(2006.01)i

       (According to International Patent Classification (IPC) or to both national classification and IPC)

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013141346 A

**Non-patent literature cited in the description**

- **TAO et al.** *Int. J. Pharm,* 2007, vol. 338, 317-326